# EUROPÄISCHE PATENTSCHRIFT

(11) **EP 0 793 545 B1**
(45) Veröffentlichungstag und Bekanntmachung des Hinweises auf die Patenterteilung: **04.08.1999**
(21) Anmeldenummer: 95938348.0
(22) Anmeldetag: 24.11.1995
(51) Int. Cl.: B08B 3/02, A47K 1/09, A61L 2/22

(54) **PFLEGESTATION FÜR ZAHNBÜRSTEN**
TOOTHBRUSH CLEANING STATION
STATION D'ENTRETIEN DE BROSSES A DENTS

(30) Priorität: 26.11.1994 DE 4442183
(43) Veröffentlichungstag der Anmeldung: 10.09.1997
(73) Patentinhaber: Hecker, Frithjof, 21614 Buxtehude (DE); Duczek, Norman, 21244 Buchholz (DE)
(72) Erfinder: Hecker, Frithjof, 21614 Buxtehude (DE); Duczek, Norman, 21244 Buchholz (DE)
(74) Vertreter: Schupfner, Gerhard D.
(86) Internationale Anmeldenummer: DE9501680
(87) Internationale Veröffentlichungsnummer: WO9616750

(56) Entgegenhaltungen:
- GB-A- 2 232 581
- US-A- 4 997 629
- US-A- 5 145 095

## Beschreibung

Die Erfindung bezieht sich auf eine Pflegestation für Zahnbürsten, in der Zahnbürstenborsten mit einem aus einer dosenförmigen Sprühvorrichtung abgebbaren desinfizierenden Medium in Berührung bringbar sind, wobei sowohl für die Sprühvorrichtung als auch für Zahnbürsten mit ihren Borsten Halterungen an einem gemeinsamen Täger vorgesehen sind, der sowohl die Zahnbürsten als auch die dosenförmige Sprühvorrichtung in ihren Halterungen getrennt voneinander, jedoch in Sprührelation zueinander haltert.

Der Mund- und Zahnpflege wird heute sehr viel Aufmerksamkeit geschenkt, um Zahnerkrankungen vorzubeugen. Dabei kommt eine ganze Reihe von Reinigungs- und Desinfektionsmitteln zum Einsatz, deren Wirkspektren alle auf die Zahnoberflächen gerichtet sind. Zum Desinfizieren von Zahnbürsten ist es aus der DE 28 02 933 A1 bekannt, deren Borsten nach ihrem Gebrauch in einen Becher zu tauchen, in dem sich eine desinfizierende Flüssigkeit befindet. Es können auch mehrere Becher nebeneinander angeordnet sein. Bei dieser Art des Desinfizierens wird sehr viel mehr Desinfektionsflüssigkeit benötigt, als zur Desinfektion an sich nötig ist.

Aus der US-A-4 997 629, welche die Merkmale des Obergriffs von Anspruch 1 aufweist, ist ein Behälter bekannt, in den eine Sprühdose einsetzbar ist. Im Kopfbereich der in den Behälter eingesetzten Sprühdose hat der Behälter eine Schulter, auf die eine gelochte Platte auflegbar ist. Durch die Löcher der Platte können ausschließlich die Stiele konventioneller Zahnbürsten gesteckt werden, deren Borstenköpfe sich dann auf der Höhe des Sprühkopfes befinden

Es ist Aufgabe der Erfindung, die Desinfektion wirkungsvoller zu gestalten und die für die Desinfektion verwendete Vorrichtung auch für eine Reise transportfähig zu machen.

Die gestellte Aufgabe ist erfindungsgemäß dadurch gelöst, daß der in die Borstenkammer mündende Sprühkopf des Ventils gegen einen Sitz des Trägers anliegt, sodaß durch Einschieben des Bodenbereiches der Sprühvorrichtung in den Träger das Ventil geöffnet wird und damit aus der Sprühvorrichtung der Sprühnebelstoß auf die Zahnbürstenborsten abgebbar ist.

Durch die Betätigung der Sprühvorrichtung von der Bodenseite her wird die Konstruktion eines Betätigungsmechanismus wesentlich einfacher. Wenn nach einer weiteren Ausgestaltung der Erfindung die eine Zahnbürste und die Sprühvorrichtung mit ihren Köpfen in der Benutzungslage nach unten angeordnet sind, dann können die Zahnpasta und das Desinfektionsmittel in der Ruhestellung nicht längs des Stieles nach unten laufen und die Pflegestation unangenehm verschmutzen. Die Zahnpasta und das Desinfektionsmittel sammeln sich am Boden unterhalb der Zahnbürste.

Hierbei ist es besonders von Vorteil, wenn der Träger eine Borstenkammer aufweist, in die ein Sprühnebelstoß der Sprühvorrichtung einführbar ist und in der sich bei in Besprühposition gebrachter Zahnbürste deren Borsten befinden, wobei nur eine Zahnbürste zur Anwendung kommt. Das Besprühen erfolgt damit in einem abgeschlossenen auf die eine zum Eisatz kommende Zahnbürste eng begrenzten Raum, in dem die Borsten dieser einen Zahnbürste verbleiben können bis die Zahnbürste erneut benutzt wird.

Mittels der Sprühvorrichtung können die Borstenbündel der einen Zahnbürste gezielt bis in die Bündelwurzeln oder Bündelfassungen desinfiziert und gereinigt werden, wozu es nur eines Sprühstrahlstoßes bedarf. Damit wird für eine sorgfältige und vollständige Desinfektion oder Reinigung nur sehr wenig Desinfektions-, Pflege- oder Reinigungsmedium benötigt.

Bei einer weiteren Ausgestaltung der Erfindung ist vorgesehen, daß die Sprühvorrichtung mittels der Halterung an den Träger anklemmbar ist. Das Anklemmen kann dabei in der Weise erfolgen, daß die Sprühvorrichtung mittels aufeinanderzu federnder Klemmarme an den Träger anklemmbar ist. Diese Art der lösbaren Anbringung ist einfach und besonders materialsparend.

Nach einer weiteren Ausgestaltung der Erfindung ist vorgesehen, daß die Zahnbürste an die Wandung des Trägers anklemmbar ist. Eine andere Art der Festlegung besteht darin, daß die Zahnbürste in einen Aufnahmeschacht an der Wandung des Trägers einsteckbar ist. Bei wieder einer anderen Art der Unterbringung ist vorgesehen, daß die Zahnbürste an die Innenseite eines Verschlußdeckels anklemmbar ist.

Nach einer weiteren Ausgestaltung der Erfindung besteht eine vorteilhafte Ausgestaltung der Unterbringung der Zahnbürste darin, daß sie in eine Mulde eines am Träger vorgesehenen Zahnbürstenetuis einlegbar ist, das mit einem aufklappbaren Verschlußdeckel verschließbar ist. Damit ist ein vorteilhaftes Reiseetui geschaffen.

Nach einer weiteren Ausgestaltung der Erfindung ist vorgesehen, daß über den Bodenbereich der Sprühvorrichtung eine Abdeckkappe setzbar ist, die den Bodenbereich gegen ein versehentliches Einschieben sichert. Dies hat eine besondere Bedeutung, wenn die Pflegestation auf Reisen mitgenommen wird.

Nach einer weiteren Ausgestaltung der Erfindung ist vorgesehen, daß die Abdeckkappe mit einer die Sprühdose in den Träger eindrückenden Auslösevorrichtung versehen ist. Eine Auslösevorrichtung in der Abdeckkappe vermindert die Gefahr einer versehentlichen Betätigung.

Nach einer weiteren Ausgestaltung der Erfindung ist vorgesehen, daß die Auslösevorrichtung aus einem Drehknopf besteht, der sich bei seinem Drehen in Richtung auf den Boden der Sprühvorrichtung derart verlängert, daß er die Sprühvorrichtung in den Träger eindrückt und damit den Sprühnebelstoß auslöst.

Nach einer weiteren Ausgestaltung der Erfindung ist vorgesehen, daß die Abdeckkappe mittels eines Bajonettverschlusses an den Träger ansetzbar ist.

Nach einer weiteren Ausgestaltung der Erfindung ist vorgesehen, daß als Sprühmedium ein bei Normaltemperaturen desinfizierendes Medium dient.

Die Erfindung wird anhand der Zeichnung näher erläutert. Es zeigen:
Fig. 1 eine Ansicht durch eine Zahnbürstenpflegestation nach der Erfindung mit einer Sprühvorrichtung, mit der die Borsten einer Zahnbürste besprüht werden können, von der Seite eines Gerätegehäuses her gesehen, an das die Zahnbürste angeklemmt ist,
Fig. 2 einen Teilschnitt durch die Zahnbürstenpflegestation,
Fig. 3 eine andere Ausführungsform der Zahnbürstenpflegestation in Seitenansicht, bei der die Zahnbürste in einer Art Tasche in ihre Pflegeposition eingebracht ist,
Fig. 4 einen Teilschnitt durch die Zahnbürstenpflegestation nach Fig. 3,
Fig. 5 eine besondere Ausgestaltung einer mit Bajonettverschluß versehenen Abdeckkappe,
Fig. 6 eine weitere Ausführungsform der Zahnpflegestation mit auf den Kopf gestellter Zahnbürste und Sprühvorrichtung, wobei die Zahnbürste in einer Verschlußkappe angeordnet ist,
Fig. 7 eine weitere Ausführungsform, bei der die Sprühvorrichtung nur seitlich angeklammert ist und sich die Zahnbürste in einem zum Träger der Zahnstation gehörenden Etui befindet,
Fig. 8 eine Draufsicht auf die Ausführungsform nach Fig. 7.

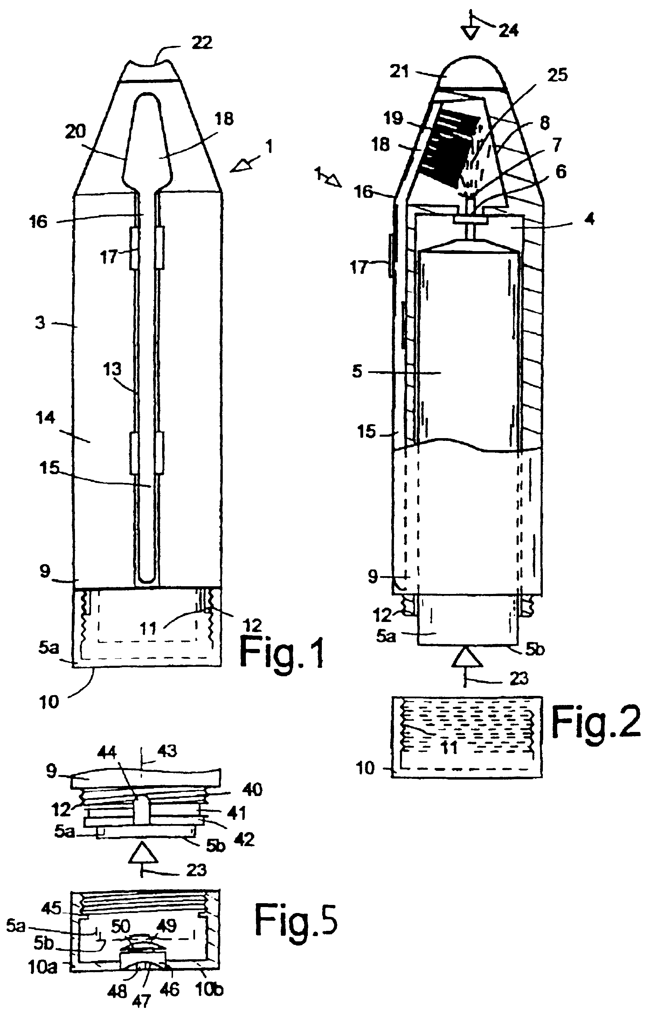

In Fig. 1 ist eine Zahnbürstenpflegestation 1 dargestellt. Diese Zahnbürstenpflegestation 1 besteht aus einem Träger 3, der sowohl eine Sprühvorichtung 5 als auch eine Zahnbürste 16 lösbar haltert. Die Halterung einer dosenförmig ausgebildeten Sprühvorrichtung 5 besteht aus einem Hohlraum 4, in den eine Sprühvorrichtung 5 einsetzbar ist. Diese Sprühvorrichtung 5 besteht aus einer Sprühdose, die mit oder ohne Druckgas arbeiten kann. Diese Sprühdose 5 ist dazu mit einem Ventil 6 ausgerüstet, dessen Sprühöffnung in eine Borstenkammer 8 des Trägers 3 hineinreicht.

Der Träger 3 ist an seinem von der Borstenkammer 8 abliegenden Ende 9 offen, sodaß die Sprühdose 5 mit ihrem Bodenteil 5a aus diesem Ende 9 herausragen kann.

Um ein versehentliches Betätigen der Sprühdose zu verhindern, ist, wie Fig. 1 zeigt, bei einem Transport über den Bodenteil 5a eine Reiseblockierkappe 10 gesetzt. Diese Reiseblockierkappe 10 hat zu ihrem Festsetzen ein Innengewinde 11, das auf ein aus dem Ende 9 des Trägers 3 herausragendes Außengewinde 12 aufschraubbar ist.

In eine Nut 13 an der Wandung 14 des Trägers 3 ist der Stiel 15 einer Zahnbürste 16 einlegbar. In der Nut 15 wird die Zahnbürste 16 mittels Klemmlaschen 17 festgeklemmt. Der Kopfteil 18 der Zahnbürste 16, oder auch nur die an ihm vorgesehenen Borsten 19, ragen durch eine Öffnung 20 in die Borstenkammer 8 hinein. Die Borstenkammer 8 ist damit bis auf die Öffnung 20 für die Borsten 19 und das Ventil 6 allseitig geschlossen. Am Kopf 21 des Trägers 3 befindet sich eine Fingermulde 22 zur Erleichterung der Betätigung.

In Fig.2 sind zwei gegeneinander gerichtete Pfeile 23 und 24 dargestellt, mit denen die Betätigungsweise der Zahnbürstenpflegestation angedeutet wird. Wird das Bodenteil 5a der Sprühdose 5 auf eine Auflage aufgesetzt oder anderweitig festgehalten, was der Pfeil 23 andeutet, und auf den Kopf 21, bzw. die Fingermulde 22 gedrückt ( Pfeil 24), dann wird das Ventil 6 geöffnet. Damit tritt ein Sprühnebelstoß 25 aus dem Ventil 6 aus, strömt in die Borstenkammer 8 und benetzt die Borsten 19. Der Nebel des auf die Borsten gestrahlten Mediums kann keimtötender Natur, also ein Antiseptikum, und/oder ein Desodorant, vorzugsweise mit aromatischen Duftstoffen sein. Die Abgabe des Mediums kann mit einem Druckgas oder unter Pumpwirkung erfolgen.

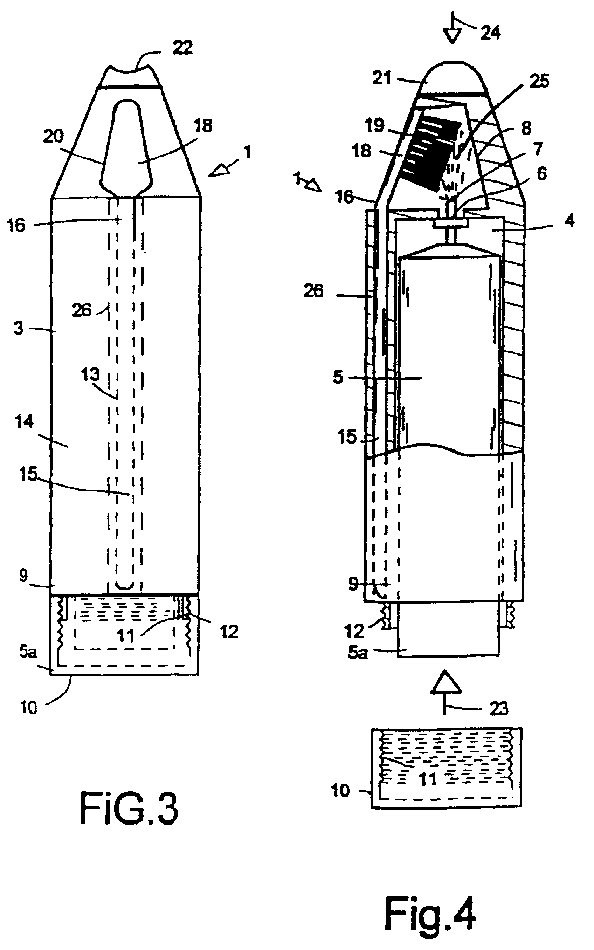

Die Fig. 3 und 4 zeigen eine andere Gehäusegestaltung. Der Stil 15 der Zahnbürste wird dabei in einen Aufnahmeschacht 26 des Trägers 3 eingesteckt. Die Zahnbürste 16 ist so insbesondere auf einer Reise gut gegen ein versehentliches Abfallen gesichert.

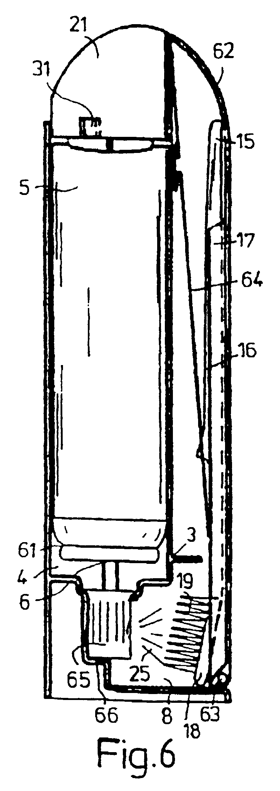

Fig.5 zeigt eine andere Ausgestaltung der Abdeckkappe 10 nach den Fig.1 bis 4 in Verbindung mit einer Abwandlung des Einschubendes 9 des Gehäuses 3. An einige Windungen 12 des Gewindeteiles 40 schließen sich eine Nut 41 und ein Endflansch 42 an. Weiterhin sind in dem Gewindeteil 40 diametral gegenüberliegende, in Längsrichtung 43 verlaufende Verschiebenuten 44 vorgesehen. Die dazu gehörige Abdeckkappe 10a hat wieder das Gewinde 11 und zusätzlich Nasen 45, die diametral aufeinanderzu weisen.

Im Boden 10b der Abdeckkappe 10a ist ein Drehknopf 46 vorgesehen, der eine Einwölbung 47 und einen Griffsteg 48 aufweist. Durch Drehen des Drehknopfes 46 kann die Oberseite 49 eines Sicherungsknopfes 50 gegen die Bodenfläche 5b geschoben und das Ventil der Sprühdose 5 damit geöffnet werden.

Der Aufbau der Abdeckkappe 10a dient der Sicherung der Sprühdose 5 in dem Gehäuse 3. Gänzlich aufgeschraubt sitzt die Abdeckkappe 10a auf dem Gewindeteil 40 fest und bietet Schutz gegen ein versehentliches Betätigen der Sprühdose 5. Durch ein Drehen der Abdeckkappe 10a gelangen die Nasen 45 in die Längsnuten 44. Die Abdeckkappe 10a kann so abgenommen werden. Wird bei aufgeschraubter Abdeckkappe 10a der Drehknopf 46 gedreht und dabei die Sicherungskappe 50 angehoben, dann wird ein Sprühnebelstoß 25 ausgelöst.

Fig.6 zeigt eine Abwandlung der Zahnpflegestation, bei der sich der Kopfteil 18 der Zahnbürste 16 und der Kopf 61 der Sprühdose 6 unten befinden. Die Sprühdose sitzt in dem Aufnahmehohlraum 4, und die Zahnbürste 16 befindet sich in einem Verschlußdeckel 62, der um ein am Träger 3 vorgesehenes Gelenk 63 schwenkbar ist. Klemmbacken 17 halten die Zahnbürste 16 lösbar fest. Es ist eine Öffnungsfeder 64 vorgesehen, die das Öffnen des Verschlußdeckel 62 erleichtert. Der Sprühkopf 65 des Ventils 6 sitzt in einem Sitz 66 an der Borstenkammer 8. Der Sprühnebel 25 kann so gezielt auf die Borsten 19 der Zahnbürste 16 geleitet werden.

Das Auslösen des Sprühnebels 25 erfolgt dann, wenn durch ein Niederdrucken der Taste 31 am Trägerkopf 21 die Sprühdose 5 nach unten gedrückt wird.

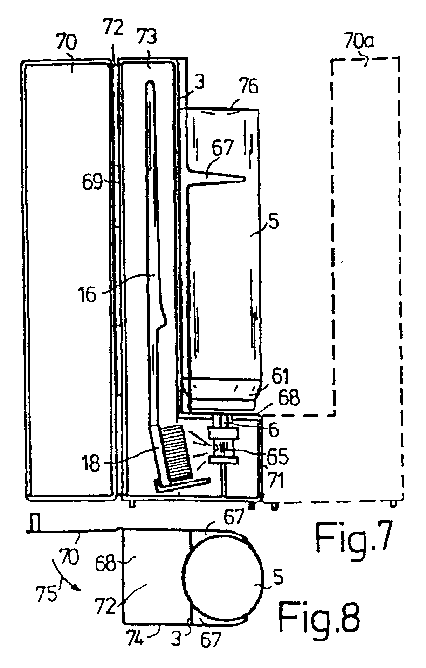

Fig. 7 zeigt eine Variante der Zahnpflegestation mit auf dem Kopf stehender Zahnbürste 16 und Sprühdose 5. Bei dieser Ausführungsform liegt die Sprühdose 5 am Träger 3 frei. Der Träger 3 ist mit Klammeramen 67 versehen, die die Sprühdose 5 umklammern. Die Wirkungsweise der Klammerarme 67 ist besonders gut aus Fig.8 zu erkennen. Das Ventil 6 der Sprühdose 5 sitzt auf einer Wand 68 der Borstenkammer 8 auf und greift durch diese Wand 68 in die Borstenkammer 8 hinein. In der Borstenkammer 8 steckt auf dem Ventil 6 der Sprühkopf 65. Integraler Bestandteil des Trägers 3 ist eine Etuikammer 73 eines Zahnbürstenetuis 72. In die Etuikammer 73 ist die Zahnbürste 16 so einlegbar, daß ihre Borsten 19 gegenüber dem Sprühkopf 65 in den Sprühbereich gelangen. In Fig. 7 ist die Vorderwand der Etuikammer 73 der besseren Übersicht wegen entfernt. Aus Fig. 8 ist diese Wand 74 zu erkennen. Die Etuikammer 68 ist über Filmgelenke 69 mit einem Etuideckel 70 verbunden. Beim Zuklappen dieses Etuideckels 70 in Richtung eines Pfeiles 75 nach Fig. 8 wird das Etui geschlossen.

In gestrichelter Darstellung ist eine Deckelvariante 70a angedeutet. Bei dieser Variante sind die Etuikammer 68 und die Borstenkammer 8 nach vorn offen. Die Deckelvariante 70a ist an eine Wand 71 der Borstenkammer 8 mittels eines Filmgelenkes angesetzt. Beim Zuklappen der Deckelvariante 70a werden sowohl die Etuikammer 68 als auch die Borstenkammer 8 geschlossen.

Die Sprühdose 5 wird betätigt, indem auf deren Boden 76 gedrückt wird.

## Patentansprüche

1. Pflegestation für Zahnbürsten, in der Zahnbürstenborsten (19) mit einem aus einer dosenförmigen Sprühvorrichtung (5) abgebbaren desinfizierenden Medium in Berührung bringbar sind, wobei sowohl für die Sprühvorrichtung (5) als auch für Zahnbürsten (16) mit ihren Borsten (19) Halterungen (4,67) an einem gemeinsamen Täger (3) vorgesehen sind, der sowohl die Zahnbürsten (16) als auch die dosenförmige Sprühvorrichtung (5) in ihren Halterungen (4,67) getrennt voneinander, jedoch in Sprührelation zueinander haltert, dadurch gekennzeichnet, daß der in die Borstenkammer mündende Sprühkopf (65) des Ventils (6) gegen einen Sitz (66) des Trägers (3) anliegt, so daß durch Einschieben des Bodenbereiches (5a,76) der Sprühvorrichtung (5) in den Träger (3) das Ventil (6) geöffnet wird und damit aus der Sprühvorrichtung (5) der Sprühnebelstoß (25) auf die Zahnbürstenborsten (19) einer Zahnbürste (16) abgebbar ist.

2. Pflegestation nach Anspruch 1, dadurch gekennzeichnet, daß die Zahnbürste (16) und die Sprühvorrichtung (5) mit ihren Köpfen nach unten angeordnet sind.

3. Pflegestation nach Anspruch 1, dadurch gekennzeichnet, daß der Träger (3) eine Borstenkammer (8) aufweist, in die ein Sprühnebelstoß (25) der Sprühvorrichtung (5) einleitbar ist und in der sich bei in Besprühposition gebrachter Zahnbürste (16) deren Borsten (19) befinden, wobei nur eine Zahnbürste (16) zu Anwendung kommt.

4. Pflegestation nach Anspruch 1, dadurch gekennzeichnet, daß die Sprühvorrichtung (5) mittels der Halterung (67) an den Träger (3) anklemmbar ist.

5. Pflegestation nach Anspruche 4, dadurch gekennzeichnet, daß die Sprühvorrichtung (5) mittels aufeinanderzu federnder Klemmarme (67) an den Träger (3) anklemmbar ist.

6. Pflegestation nach Anspruch 4, dadurch gekennzeichnet, daß die Zahnbürste (16) an die Wandung (14) des Trägers (3) anklemmbar ist.

7. Pflegestation nach Anspruch 1, dadurch gekennzeichnet, daß die Zahnbürste (16) in einen Aufnahmeschacht an der Wandung (14) des Trägers (3) einsteckbar ist.

8. Pflegestation nach Anspruch 1, dadurch gekennzeichnet, daß die Zahnbürste (16) an die Innenseite eines Verschlußdeckels (62) anklemmbar ist.

9. Pflegestätion nach Anspruche 1 und/oder 7, dadurch gekennzeichnet, daß die Zahnbürste (16) in eine Mulde (68) eines am Träger (3) vorgesehenen Zahnbürstenetuis (72) einlegbar ist, das mit einem aufklappbaren Deckel (70,70a) verschließbar ist.

10. Pflegestation nach einem oder mehreren der Ansprüche 1 bis 9, dadurch gekennzeichnet, daß über den Bodenbereich (5a) der Sprühvorrichtung (5) eine Abdeckkappe (10,10a) setzbar ist, die den Bodenbereich (5a) gegen ein versehentliches Einschieben sichert.

11. Pflegestation nach Anspruch 10, dadurch gekennzeichnet, daß die Abdeckkappe (10a) mit einer die Sprühvorrichtung (5) in den Träger (3) eindrückenden Auslösevorrichtung (46) versehen ist.

12. Pflegestation nach Anspruch 11, dadurch gekennzeichnet, daß die Auslösevorrichtung (46) aus einem Drehknopf besteht, der sich bei seinem Drehen in Richtung auf den Boden (5b) der Sprühvorrichtung (5) derart verlängert, daß er die Sprühvorrichtung (5) in den Träger (3) eindrückt und damit den Sprühnebelstoß (25) auslöst.

13. Pflegestation nach Anspruch 11, dadurch gekennzeichnet, daß die Abdeckkappe (10a) mittels eines Bajonettverschlusses (41,44,45) an den Träger (3) ansetzbar ist.

14. Pflegestation nach einem oder mehreren der Ansprüche 1 bis 13, dadurch gekennzeichnet, daß als Sprühmedium ein bei Normaltemperaturen desinfizierendes Medium dient.

## Claims

1. Care station for toothbrushes, in which toothbrush bristles (19) can be contacted with a disinfectant medium that can be released from a can-shaped spraying device (5), whereby holding devices (4, 67) for the spraying device (5) as well as for toothbrushes (16) with their bristles (19) are provided on a common support (3), which fastens both the toothbrushes (16) and the can-shaped spraying device (5) in their holding devices (4, 67) separatly from each other, yet in spraying relation to each other, **characterized in that** the spray head (65) of the valve (6) projecting into the bristle chamber fits against a seat (66) of the support (3), so that by clamping the bottom part (5a, 76) of the spraying device (5) into the support (3) the valve (6) is opened and therewith from the spraying device (5) the spray mist (25) can be released on to the toothbrush bristles (19) of a toothbrush (16).

2. Care station as claimed in claim 1, characterized in that the toothbrush (16) and the spraying device (5) are arranged with their heads pointed downwards.

3. Care station as claimed in claim 1, characterized in that the support (3) has a bristle chamber (8), into which a push of a spray mist (25) of the spraying device (5) can be introduced and in which the bristles (19) of the toothbrush (16) are situated, when the toothbrush (16) is positioned in the spraying position, only one toothbrush (16) being used.

4. Care station as claimed in claim 1, characterized in that the spraying device (5) can be clamped to the support (3) by means of the holding device (67).

5. Care station as claimed in claim 4, characterized in that the spraying device (5) can be clamped to the support (3) by means of clamping arms (67) springing towards each other.

6. Care station as claimed in claim 4, characterized in that the toothbrush (16) can be clamped to the wall (14) of the support (3).

7. Care station as claimed in claim 1, characterized in that the toothbrush (16) can be inserted into a receiving shaft at the wall (14) of the support (3).

8. Care station as claimed in claim 1, characterized in that the toothbrush (16) can be clamped to the inner side of a closing cap (62).

9. Care station as claimed in claim 1 and/or 7, characterized in that the toothbrush (16) can be placed in a depression (68) of a toothbrush case (72) provided on the support (3), which case is closable with a hinged cap (70,70a).

10. Care station as claimed in one or several of claims 1 to 9, characterized in that a covering cap (10,10a) can be placed over the bottom region (5a) of the spraying device (5), which secures the bottom region (5a) against unintentional pushing in.

11. Care station as claimed in claim 10, characterized in that the covering cap (10a) is equipped with a release mechanism (46) pressing the spraying device (5) into the support (3).

12. Care station as claimed in 11, characterized in that the release mechanism (46) consists of a turning knob, which upon turning is extended in the direction of the bottom (5b) of the spraying device (5) such that it pushes the spraying device (5) into the support (3) and with this actuates the push of the spray mist (25).

13. Care station as claimed in claim 11, characterized in that the covering cap (10a) can be attached to the support (3) by means of a bayonet joint (41,44,45).

14. Care station as claimed in one or several of claims 1 to 13, characterized in that a medium disinfecting at normal temperatures serves as a spray medium.

## Revendications

1. Appareil d'entretien pour brosses à dents, dans lequel les soies (19) de la brosse à dents peuvent être mises en contact avec un fluide désinfectant pouvant être distribué par un dispositif de pulvérisation (5) en forme de boîte métallique, des attaches (4, 67) destinées aussi bien au dispositif de pulvérisation (5) qu'aux brosses à dents (16) avec leurs soies (19) étant prévues sur un support commun (3) qui maintient aussi bien les brosses à dents (16) que le dispositif de pulvérisation en forme de boîte (5) dans leurs attaches (4, 67) de manière qu'ils soient séparés mutuellement, néanmoins en relation de pulvérisation, caractérisé en ce que la tête de pulvérisation (65) de la valve (6), qui débouche dans la chambre à soies, est en appui contre un siège (66) du support (3), de sorte que l'insertion de la partie de fond (5a, 76) du dispositif de pulvérisation (5) dans le support (3) ouvre la valve (6) et donc un jet de brouillard de pulvérisation (25) peut être projeté par le dispositif de pulvérisation (5) sur les soies (19) d'une brosse à dents (16).

2. Appareil d'entretien selon la revendication 1, caractérisé en ce que la tête de la brosse à dents (16) ainsi que celle du dispositif de pulvérisation (5) sont disposées vers le bas.

3. Appareil d'entretien selon la revendication 1, caractérisé en ce que le support (3) comporte une chambre (8) prévue pour les soies et dans laquelle un jet de brouillard de pulvérisation (25) provenant du dispositif de pulvérisation (5) peut être dirigé et dans laquelle se trouvent les soies (19) de la brosse à dents (16) mise en position d'aspersion, seule une brosse à dents (16) étant mise en application.

4. Appareil d'entretien selon la revendication 1, caractérisé en ce que le dispositif de pulvérisation (5) peut être monté par serrage sur le support (3) au moyen de l'attache (67).

5. Appareil d'entretien selon la revendication 4, caractérisé en ce que le dispositif de pulvérisation (5) peut être monté par serrage sur le support (3) au moyen de bras de serrage (67) faisant ressort l'un contre l'autre.

6. Appareil d'entretien selon la revendication 4, caractérisé en ce que la brosse à dents (16) peut être montée par serrage sur la paroi (14) du support (3).

7. Appareil d'entretien selon la revendication 1, caractérisé en ce que la brosse à dents (16) peut être insérée dans une colonne de logement contre la paroi (14) du support (3).

8. Appareil d'entretien selon la revendication 1, caractérisé en ce que la brosse à dents (16) peut être montée par serrage contre le côté intérieur d'un couvercle obturateur (62).

9. Appareil d'entretien selon la revendication 1 et/ou 7, caractérisé en ce que la brosse à dents (16) peut être placée dans une cavité (68) d'un étui (72) de brosse à dents qui est prévu sur le support (3) et qui est obturable par un couvercle rabattable (70, 70a).

10. Appareil d'entretien selon l'une ou plusieurs des revendications 1 à 9, caractérisé en ce qu'un capuchon de couverture (10, 10a), qui peut être monté sur la partie de fond (5a) du dispositif de pulvérisation (5), offre une sécurité empêchant que la partie de fond (5a) soit insérée de manière erronée.

11. Appareil d'entretien selon la revendication 10, caractérisé en ce que le capuchon de couverture (10a) est équipé d'un dispositif de déclenchement (46) qui repousse le dispositif de pulvérisation (5) dans le support (3).

12. Appareil d'entretien selon la revendication 11, caractérisé en ce que le dispositif de déclenchement (46) consiste en un bouton rotatif qui, lors de sa rotation le rapprochant du fond (5b) du dispositif de pulvérisation (5), s'allonge de manière qu'il repousse le dispositif de pulvérisation (5) dans le support (3) et ainsi déclenche le jet de brouillard de pulvérisation (25).

13. Appareil d'entretien selon la revendication 11, caractérisé en ce que le capuchon obturateur (10a) peut se monter sur le support (3) au moyen d'une fermeture à baïonnette (41, 44, 45).

14. Appareil d'entretien selon l'une ou plusieurs des revendications 1 à 13, caractérisé en ce qu'un fluide désinfectant aux températures normales sert de fluide de pulvérisation.
